# FASCICULE DE BREVET EUROPEEN

(11) **EP 1 920 759 B1**
(45) Date de publication et mention de la délivrance du brevet: **11.05.2016**
(21) Numéro de dépôt: 07120274.1
(22) Date de dépôt: 08.11.2007
(51) Int. Cl.: A61K 8/39, A61K 8/55, A61Q 1/10

(54) **Composition cosmétique comprenant un phosphate d'alkyle et un ether d'alcool gras et de polyethylèneglycol, procedes et utilisations**
Kosmetische Zusammensetzung, die ein Alkylphosphat, einen Fettalkohol-Ester und eine Polyethylenglycol-Ester umfasst, Herstellungsverfahren und Anwendungen
Cosmetic composition comprising an alkyl phosphate and a polyethylene glycol and fatty alcohol ester, methods and uses.

(30) Priorité: 10.11.2006 FR 0654827
(43) Date de publication de la demande: 14.05.2008
(73) Titulaire: L'Oréal, 75008 Paris (FR)
(72) Inventeur: Raineau, Olivier, 75013 Paris (FR); Jacquier, Isabelle, 94550, Chevilly Larue (FR)
(74) Mandataire: Eveillard, Sophie

(56) Documents cités:
- EP-A- 1 529 513
- WO-A-02/098379
- WO-A-2005/039514
- WO-A1-2008/015272

## Description

La présente demande se rapporte au domaine du maquillage ou du soin des fibres kératiniques comme les cils ou les sourcils, plus particulièrement elle se rapporte à des compositions de revêtement des cils ou mascara.

Les compositions de revêtement des cils tels que les mascaras sont généralement des compositions de maquillage, des compositions à appliquer sur un maquillage (encore appelées « top-coat »), ou encore des compositions de soin cosmétique des cils.

Les mascaras sont notamment préparés selon deux types de formulation : les mascaras aqueux dits mascaras crèmes, sous forme de dispersion de cires dans l'eau ; les mascaras anhydres ou à faible teneur en eau, dits mascaras water-proofs, sous forme de dispersions de cires dans des solvants organiques.

La présente demande concerne plus spécifiquement les mascaras aqueux.

L'application de mascara permet d'augmenter le volume des cils et en conséquence d'augmenter l'intensité du regard. Pour cela, il existe de nombreux mascaras épaississants ou volumateurs dont le principe consiste à déposer le maximum de matière sur les cils de manière à obtenir un effet volumateur (ou chargeant).

C'est en particulier à travers la quantité de particules solides (notamment les cires, qui permettent de structurer la composition), que peuvent être ajustées les spécificités d'application recherchées pour les compositions, comme par exemple leur fluidité ou consistance, ainsi que leur pouvoir épaississant (encore appelé pouvoir chargeant ou maquillant).
Ces particules solides sont dispersées dans le mascara crème à l'aide d'un système tensioactif.

Parmi les émulsionnants ou systèmes émulsionnant classiques, on compte :
- le phosphate de cétyle, cependant l'utilisation du phosphate de cétyle seul conduit à une agglomération des pigments ainsi qu'à une dispersion grossière des cires ; ceci résultant fréquemment en un mascara « gris » et non noir ;
- les systèmes émulsionnants à base de steareth-20 et de steareth-2, cependant l'utilisation de ces systèmes mène à des mascaras très fluides dont la consistance est non satisfaisante pour un mascara volumateur ;
- les systèmes émulsionnants à base de stéarate de triéthanolamine.

WO 2005/039514 décrit un mascara contenant au moins du phosphate de dicétyle, du stéareth-2 et du stéareth-10 comme émulsifiants et une pâte pigmentaire.

Le problème posé dans la présente demande est de proposer un mascara dans lequel non seulement les cires mais aussi les pigments sont dispersés de façon homogène, ledit mascara présentant une texture suffisamment épaisse pour obtenir un dépôt chargeant, volumateur sur les cils, et présentant une consistance satisfaisante permettant une application facile sur les cils et un dépôt lisse et homogène.

De manière surprenante et inattendue, les inventeurs de la présente demande ont résolu ce problème au moyen d'un système émulsionnant contenant au moins un phosphate d'alkyle en C₁₀-C₃₀ et et au moins un éther d'alcool gras en C₈-C₂₄ et de polyéthylène glycol, ledit éther comprenant de 1 à 19 motifs d'éthylèneglycol et présentant une HLB< 8 à 25°C

Les inventeurs de la présente demande ont pu observer que le système émulsionnant défini dans la présente demande permet une bonne dispersion des pigments et/ou des cires, cette dispersion est de la qualité de celles obtenues avec les systèmes émulsionnants à base de stéarate de triéthanolamine. Cette composition permet d'obtenir un maquillage chargeant des fibres kératiniques et un dépôt lisse et homogène sur lesdites fibres.

La composition selon l'invention présente avantageusement une viscosité allant de 1 à 60 Pa.s, de préférence de 1,5 à 50 Pa.s, mieux de 2 à 40 Pa.s et encore mieux de 3 à 30 Pa.s.

La viscosité de la composition est mesurée à 25°C à l'aide d'un Rhéomat 180 (Société LAMY) équipé d'un mobile MS-R1, MS-R2, MS-R3, MS-R4 ou MS-R5 choisi en fonction de la consistance de la composition, tournant à une vitesse de rotation de 200 t.min-1. La mesure est prise après 10 min de rotation.

Un premier objet de la présente demande est une composition cosmétique de revêtement des fibres kératiniques comprenant une phase aqueuse et un système émulsionnant tel que le système émulsionnant contient :
- au moins un phosphate d'alkyle en C₁₀-C₃₀ et
- au moins un éther d'alcool gras en C₈-C₂₄ et de polyéthylène glycol, ledit éther comprenant de 1 à 19 motifs d'éthylèneglycol et présentant une HLB< 8 à 25°C, et
- au moins une matière colorante.

La balance HLB (hydrophile-lipophile balance) au sens de GRIFFIN est définie dans J. Soc. Cosm. Chem. 1954 (volume 5), pages 249-256.

Un deuxième objet de la présente demande est un procédé de maquillage ou de soin non thérapeutique des fibres kératiniques comprenant l'application sur lesdites fibres kératiniques de la composition selon la présente demande.

Un troisième objet de la présente demande concerne les utilisations de la composition selon la présente demande, en particulier l'utilisation de cette composition pour obtenir un maquillage homogène et/ou volumateur des cils.

D'autres caractéristiques, propriétés et avantages de la présente invention apparaîtront plus clairement à la lecture de la description et des exemples qui suivent.

### Système émulsionnant

Le ou les phosphate(s) d'alkyle utilisable(s) dans les compositions selon la présente demande est/sont de préférence choisi(s) parmi les phosphates d'alkyle en C14-C24, de préférence en C₁₆-C₁₈ et leurs mélanges. De manière encore préférée, ils sont choisi parmi le phosphate de cétyle, le phosphate de stéaryle et le phosphate de cétéaryle. De manière particulière, il s'agit du phosphate de cétyle, par exemple commercialisé sous les dénominations Amphisol K (Roche), Amphisol A (Roche), Arlatone MAP (Uniqema), crodafos MCA (Croda).

La teneur en phosphate(s) d'alkyle va de 0,1 à 20% en poids, de préférence de 0,5 à 12% en poids par rapport au poids total de la composition.

Le ou les éther(s) d'alcool gras en C₈-C₂₄ et de polyéthylène glycol comprenant de 1 à 19 motifs d'éthylèneglycol, et présentant une HLB< 8 à 25°C utilisable(s) dans les compositions selon la présente demande est/sont de préférence choisis parmi les éthers d'alcools gras en C₁₆-C₂₂ et de polyéthylène glycol comprenant de 1 à 19 motifs d'éthylèneglycol

De manière particulière il s'agit du composé portant le nom INCI steareth-2 (polyethyleneglycol(2)stearyl ether) de HLB égale à 5, ce produit est par exemple commercialisé sous les dénominations Brij 72 (Uniqema), Volpo S2 (Croda).

La teneur en éther d'alcool gras en C₈-C₂₄ et de polyéthylène glycol, comprenant de 1 à 19 motifs d'éthylèneglycol et de HLB< 8 à 25°C va de 0,1 à 20% en poids, par rapport au poids total de la composition, de préférence de 0,5 à 10% en poids.

Selon un mode de réalisation, le système émulsionnant contenant l'association de phosphate d'alkyle en C10-C30 et d'éther d'alcool gras en C8-C24 et de polyéthylène glycol, ledit éther comprenant de 1 à 19 motifs d'éthylèneglycol et présentant une HLB< 8 à 25°C, constitue le système tensioactif principal de la composition.

Par « système tensioactif principal », on entend un système qui, en son absence, ne conduit pas à la formation d'une composition stable.

Par « stable », on entend une composition qui, après avoir été placée dans une étuve à 45°C pendant deux mois, ne présente pas, après retour à température ambiante, de grains perceptibles au toucher, lorsqu'une couche fine de la composition est cisaillée entre les doigts.

Selon un mode de réalisation particulier, le système émulsionnant contenant l'association de phosphate d'alkyle en C10-C30 et d'éther d'alcool gras en C8-C24 et de polyéthylène glycol, ledit éther comprenant de 1 à 19 motifs d'éthylèneglycol et présentant une HLB< 8 à 25°C, constitue l'unique système tensioactif de la composition.

Par « unique », on entend que tout éventuel système tensioactif additionnel est présent en une teneur n'excédant pas 1%, et de préférence n'excédant pas 0,5%. De préférence encore, par « unique », on désigne une absence totale de tout autre système tensioactif.

Selon une variante, la composition cosmétique selon la présente demande comprend moins de 1%, de préférence moins de 0,5% en poids de triéthanolamine, et mieux, est exempte de triéthanolamine.

Selon une variante préférée, la composition cosmétique selon la présente demande comprend moins de 1%, de préférence moins de 0,5% en poids de stéarate de triéthanolamine, et mieux, est exempte de stéarate de triéthanolamine.

La composition selon l'invention comprend bien entendu un milieu physiologiquement acceptable. Par « composé ou milieu physiologiquement acceptable » au sens de la présente demande, on entend un composé ou milieu dont l'utilisation est compatible avec une application sur les cils.

### Phase aqueuse

La composition selon l'invention comprend une phase aqueuse, qui peut former la phase continue de la composition.

Par composition à phase continue aqueuse, on entend que la composition présente une conductivité, mesurée à 25 °C, supérieure ou égale à 23 µS/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.

La phase aqueuse comprend de l'eau et/ou au moins un solvant hydrosoluble.

Par "solvant hydrosoluble", on désigne dans la présente invention un composé liquide à température ambiante et miscible à l'eau (miscibilité dans l'eau supérieure à 50 % en poids à 25 °C et pression atmosphérique).

Les solvants hydrosolubles utilisables dans les compositions selon l'invention peuvent en outre être volatils.

Parmi les solvants hydrosolubles pouvant être utilisés dans les compositions conformes à l'invention, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol.

La phase aqueuse (eau et éventuellement le solvant miscible à l'eau) est généralement présente dans la composition selon la présente demande en une teneur allant de 1 % à 95 % en poids, par rapport au poids total de la composition, de préférence allant de 3 % à 80 % en poids, et préférentiellement allant de 5 % à 60 % en poids.

Le système émulsionnant peut en outre contenir au moins un agent tensioactif additionnel, différent des phosphates d'alkyle en C₁₀-C₃₀ et de l'éther d'alcool gras et de polyéthylène glycol tel que défini ci-dessus, choisi de manière appropriée pour l'obtention d'une émulsion cire dans eau ou huile-dans-eau.

Selon la présente invention, l'agent tensioactif additionnel n'est pas un système tensioactif tel que défini ci-dessus, étant donné que cet agent tensioactif additionnel seul ne peut conduire à la formation d'une composition stable, tel que définie ci-dessus.

En particulier, on peut utiliser un émulsionnant possédant à 25 C une balance HLB (hydrophile-lipophile balance) au sens de GRIFFIN, supérieure ou égale à 8.

Ces agents tensioactifs additionnels peuvent être choisis parmi les agents tensioactifs non ioniques, anioniques, cationiques, amphotères ou encore les émulsionnants tensioactifs. On peut se reporter au document "Encyclopedia of Chemical Technology, KIRK-OTHMER", volume 22, p.333-432, 3ème édition, 1979, WILEY, pour la définition des propriétés et des fonctions (émulsionnant) des tensioactifs, en particulier p. 347-377 de cette référence, pour les tensioactifs anioniques, amphotères et non ioniques.

Ces tensioactifs additionnels peuvent être préférentiellement choisis parmi :
a) les agents tensioactifs non ioniques de HLB supérieur ou égal à 8 à 25 °C, utilisés seuls ou en mélange; on peut citer notamment :
   - les éthers oxyéthylénés et/ou oxypropylénés (pouvant comporter de 1 à 150 groupes oxyéthylénés et/ou oxypropylénés) de glycérol ;
   - les éthers oxyéthylénés et/ou oxypropylénés (pouvant comporter de 20 à 1000 groupes oxyéthylénés et/ou oxypropylénés) d'alcools gras (notamment d'alcoolen C₈-C₂₄, et de préférence en C₁₂-C₁₈) tels que l'éther oxyéthyléné de l'alcool cétéarylique à 30 groupes oxyéthylénés (nom CTFA "Ceteareth-30 "), l'éther oxyéthyléné de l'alcool stéarylique à 20 groupes oxyéthylénés (nom CTFA "Steareth-20 ") tel que le BRIJ 78 commercialisé par la société UNIQEMA, l'éther oxyéthyléné de l'alcool cétéarylique à 30 groupes oxyéthylénés (nom CTFA "Ceteareth-30 ") et l'éther oxyéthyléné du mélange d'alcools gras en C12-C15 comportant 7 groupes oxyéthylénés (nom CTFA "C12-15 Pareth-7" commercialisé sous la dénomination NEODOL 25-7® par SHELL CHEMICALS,
   - les esters d'acide gras (notamment d'acide en C₈-C₂₄, et de préférence en C₁₆-C₂₂) et de polyéthylène glycol (pouvant comprendre de 1 à 150 motifs d'éthylèneglycol) tels que le stéarate de PEG-50 et le monostéarate de PEG-40 commercialisé sous le nom MYRJ 52P^{Ⓡ} par la société ICI UNIQEMA,
   - les esters d'acide gras (notamment d'acide en C8-C24, et de préférence en C16-C22) et des éthers de glycérol oxyéthylénés et/ou oxypropylénés (pouvant comporter de 1 à 150 groupes oxyéthylénés et/ou oxypropylénés), comme le monostéarate de PEG-200 glycéryle vendu sous la dénomination Simulsol 220 TM^{®} par la société SEPPIC ; le stéarate de glycéryle polyéthoxylé à 30 groupes d'oxyde d'éthylène comme le produit TAGAT S^{®} vendu par la société GOLDSCHMIDT, l'oléate de glycéryle polyéthoxylé à 30 groupes d'oxyde d'éthylène comme le produit TAGAT O^{®} vendu par la société GOLDSCHMIDT, le cocoate de glycéryle polyéthoxylé à 30 groupes d'oxyde d'éthylène comme le produit VARIONIC LI 13^{®} vendu par la société SHEREX, l'isostéarate de glycéryle polyéthoxylé à 30 groupes d'oxyde d'éthylène comme le produit TAGAT L^{®} vendu par la société GOLDSCHMIDT et le laurate de glycéryle polyéthoxylé à 30 groupes d'oxyde d'éthylène comme le produit TAGAT I^{®} de la société GOLDSCHMIDT,

   - les esters d'acide gras (notamment d'acide en C8-C24, et de préférence en C16-C22) et des éthers de sorbitol oxyéthylénés et/ou oxypropylénés (pouvant comporter de 1 à 150 groupes oxyéthylénés et/ou oxypropylénés), comme le polysorbate 60 vendu sous la dénomination Tween 60^{®} par la société UNIQUEMA,
   - la diméthicone copolyol, telle que celle vendue sous la dénomination Q2-5220^{®} par la société DOW CORNING,
   - la diméthicone copolyol benzoate (FINSOLV SLB 101^{®} et 201^{®} de la société FINTEX),
   - les copolymères d'oxyde propylène et d'oxyde d'éthylène, également appelés polycondensats OE/OP,
   - et leurs mélanges.

   Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante :

   H-(O-CH₂-CH₂)ₐ-(O-CH(CH₃)-CH₂)_{b}-(O-CH₂-CH₂)ₐ-OH,

   formule dans laquelle a va de 2 à 120, et b va de 1 à 100.
   Le polycondensat OE/OP a de préférence un poids moléculaire moyen en poids allant de 1000 à 15000, et de mieux allant de 2000 à 13000. Avantageusement, ledit polycondensat OE/OP a une température de trouble, à 10 g/l en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les polycondensats tribloc polyéthylène glycol / polypropylène glycol / polyéthylène glycol vendus sous les dénominations SYNPERONIC^{®} comme les SYNPERONIC PE/ L44^{®} et SYNPERONIC PE/F127^{®} par la société ICI.
b) les agents tensioactif non ioniques de HLB inférieur à 8 à 25 °C, éventuellement associés à un ou plusieurs agents tensioactif non ioniques de HLB supérieur à 8 à 25 °C, tels que cités ci-dessus tels que :
   - les esters et éthers d'oses tels que les stéarate de sucrose, cocoate de sucrose, stéarate de sorbitan et leurs mélanges comme l'Arlatone 2121^{®} commercialisé par la société ICI ;
   - les esters d'acides gras (notamment d'acide en C8-C24, et de préférence en C16-C22) et de polyol, notamment de glycérol ou de sorbitol, tels que stéarate de glycéryle, stéarate de glycéryle tel que le produit vendu sous la dénomination TEGIN M^{®} par la société GOLDSCHMIDT, laurate de glycéryle tel que le produit vendu sous la dénomination IMWITOR 312^{®} par la société HULS, stéarate de polyglycéryl-2, tristéarate de sorbitan, ricinoléate de glycéryle ;
   - le mélange de cyclométhicone/diméthicone copolyol vendu sous la dénomination Q2-3225C^{®} par la société DOW CORNING.
   - c) Les tensioactifs anioniques tels que :
   - les sels d'acides gras en C16-C30 notamment ceux dérivant des amines, comme le stéarate de triéthanolamine et/ou le stéarate d'amino-2-méthyl-2-propane di-ol-1,3 ; mais de préférence la composition selon la présente demande ne contient pas de stéarate de triéthanolamine.
   - les sels d'acides gras polyoxyéthylénés notamment ceux dérivant des amines ou les sels alcalins, et leurs mélanges ;
   - les alkyléthersulfates tels que le lauryl éther sulfate de sodium ;
   - les iséthionates ;
   - les acylglutamates tels que le "Disodium hydrogenated tallow glutamate" (AMISOFT HS-21 R^{®} commercialisé par la société AJINOMOTO) et leurs mélanges.

La composition conforme à l'invention peut également contenir un ou plusieurs tensioactifs amphotères comme les N-acyl-aminoacides tels que les N-alkyl-aminoacétates et le cocoamphodiacetate disodique et les oxydes d'amines tels que l'oxyde de stéaramine ou encore des tensioactifs siliconés comme les diméthicone copolyols phosphates tels que celui vendu sous la dénomination PECOSIL PS 100^{®} par la société PHOENIX CHEMICAL.

Selon un mode de réalisation particulier, la composition comprend un tensioactif additionnel de HLB > 8 à 25°C, de préférence un éther d'alcool gras en C₈-C₂₄ et de polyéthylène glycol, ledit éther comprenant de 20 à 1000 motifs d'éthylèneglycol. Ledit éther peut être présent en une teneur allant de 0,1 à 10% en poids par rapport au poids total de la composition, de préférence 0,2 à 8% en poids.

On peut citer en particulier l'éther oxyéthyléné de l'alcool stéarylique à 20 groupes oxyéthylénés (nom CTFA "Steareth-20 ").

Selon un mode de réalisation préféré, la composition comprend en outre un co-tensioactif choisi parmi les alcools gras, comprenant de préférence de 10 à 30 atomes de carbone. Par alcool gras comprenant de 10 à 30 atomes de carbone, on entend tout alcool gras pur, saturé ou non, ramifié ou non, comportant de 10 à 30 atomes de carbone.

On utilise de préférence un alcool gras comprenant de 10 à 26 atomes de carbone, mieux de 10 à 24 atomes de carbone et encore mieux de 14 à 22 atomes de carbone.

On peut citer notamment comme alcools gras utilisables dans la composition les alcools laurique, myristique, cétylique, stéarylique, oléique, cétéarylique (mélange d'alcool cétylique et stéarylique), béhénique, érucique et leurs mélanges. On utilise de préférence l'alcool cétylique.

De tels alcools gras sont notamment commercialisés sous la dénomination NAFOL par la société SASOL.

L'alcool gras peut être présent en une teneur allant de 0,2 à 20% en poids, de préférence de 0,3 à 10% en poids par rapport au poids total de la composition.

Selon un mode de réalisation, la composition selon l'invention comprend comme système émulsionnant l'association suivante :
- d'au moins un tensioactif phosphate d'alkyle en C₁₀-C₃₀,
- au moins un éther d'alcool gras en C₈-C₂₄ et de polyéthylène glycol, ledit éther comprenant de 1 à 19 motifs d'éthylèneglycol et présentant une HLB< 8 à 25°C,
- au moins un éther d'alcool gras en C₈-C₂₄ et de polyéthylène glycol, ledit éther comprenant de 20 à 1000 motifs d'éthylèneglycol et de HLB > 8 à 25°C et
- au moins un alcool gras comprenant de 10 à 30 atomes de carbone.

Dans la composition conforme à l'invention, la teneur totale en agents tensioactifs peut aller de 0,1 à 30 % en poids par rapport au poids total de la composition, de préférence de 1 à 20 % et mieux de 2 à 15 % en poids

### Cire(s)

La composition selon la présente demande comprend avantageusement au moins une cire.

Par cire au sens de la présente invention, on entend un composé lipophile, solide à température ambiante (25 °C), à changement d'état solide/liquide réversible, ayant un point de fusion supérieur ou égal à 30°C pouvant aller jusqu'à 120°C.

Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 25°C et mieux supérieure à 45°C.

La cire peut être présente en une teneur allant de 0,1 à 50 % en poids par rapport au poids total de la composition, mieux de 1 à 40 % et encore mieux de 5 à 30% en poids.

On peut notamment utiliser les cires hydrocarbonées comme la cire d'abeille, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.

On peut aussi citer les cires obtenues par hydrogénation catalytique d'huiles animales ou végétales ayant des chaînes grasses, linéaires ou ramifiées, en C8-C32.

Parmi celles-ci, on peut notamment citer l'huile de jojoba hydrogénée, l'huile de jojoba isomérisée telle que l'huile de jojoba partiellement hydrogénée isomérisée trans fabriquée ou commercialisée par la société Desert Whale sous la référence commerciale ISO-JOJOBA-50®, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination "HEST 2T-4S" par la société HETERENE, le tétrabéhénate de di-(triméthylol-1,1,1 propane) vendue sous la dénomination HEST 2T-4B par la société HETERENE.

On peut encore citer les cires de silicone comme les alkyl ou alkoxy-diméticone ayant de 16 à 45 atomes de carbone, les cires fluorées.

On peut également utiliser la cire obtenue par hydrogénation d'huile d'olive estérifiée avec l'alcool stéarylique vendue sous la dénomination "PHYTOWAX Olive 18 L 57" ou bien encore les cires obtenues par hydrogénation d'huile de ricin estérifiée avec l'alcool cétylique vendus sous la dénomination "PHYTOWAX ricin 16L64 et 22L73", par la société SOPHIM. De telles cires sont décrites dans la demande FR-A- 2792190.

Selon un mode de réalisation particulier, les compositions conformes à l'invention peuvent comprendre au moins une cire dite cire collante, c'est-à-dire possédant un collant supérieur ou égal à 0,7 N.s et une dureté inférieure ou égale à 3,5 MPa.

L'utilisation d'une cire collante peut notamment permettre l'obtention d'une composition cosmétique qui s'applique facilement sur les cils, ayant une bonne accroche sur les cils et qui conduit à la formation d'un maquillage lisse, homogène et épaississant.

La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s à 10 N.s, et en particulier allant de 2 N.s à 5 N.s.

Le collant de la cire est déterminé par la mesure de l'évolution de la force (force de compression ou force d'étirement) en fonction du temps, à 20 °C à l'aide du texturomètre vendu sous la dénomination "TA-TX2i®" par la société RHEO, équipé d'un mobile en polymère acrylique en forme de cône formant un angle de 45°.

Le protocole de mesure est le suivant :
La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.

Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.

La dureté est mesurée selon le protocole décrit précédemment.

Comme cire collante, on peut utiliser un (hydroxystéaryloxy)stéarate d'alkyle en C20-C40 (le groupe alkyle comprenant de 20 à 40 atomes de carbone), seul ou en mélange, en particulier un 12-(12'-hydroxystéaryloxy)stéarate d'alkyle en C20-C40, de formule (II) : 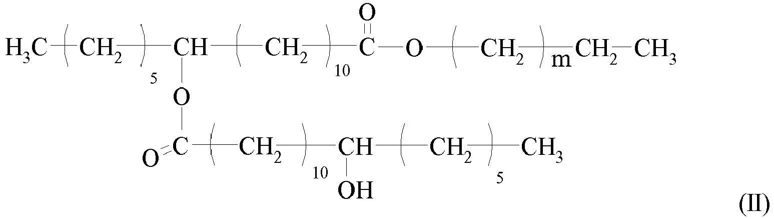 dans laquelle m est un entier allant de 18 à 38, ou un mélange de composés de formule (II).

Une telle cire est notamment vendue sous les dénominations "Kester Wax K 82 P®" et "Kester Wax K 80 P®" par la société KOSTER KEUNEN.

Les cires citées ci-dessus présentent généralement un point de fusion commençante inférieur à 45 °C.

On peut également utiliser la cire microcristalline commercialisée sous la référence SP18 par la société STRAHL and PITSCH qui présente une dureté d'environ 0,46 MPa et une valeur de collant d'environ 1 N.s.

La ou les cires peu(ven)t être présente(s) sous forme d'une microdispersion aqueuse de cire. On entend par microdispersion aqueuse de cire, une dispersion aqueuse de particules de cire, dans laquelle la taille desdites particules de cire est inférieure ou égale à environ 1 µm.

Les microdispersions de cire sont des dispersions stables de particules colloïdales de cire, et sont notamment décrites dans "Microemulsions Theory and Practice", L.M. Prince Ed., Academic Press (1977) pages 21-32.

En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.

Les microdispersions de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 µm (notamment allant de 0,02 µm à 0,99 µm), de préférence inférieures à 0,5 µm (notamment allant de 0,06 µm à 0,5 µm).

Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

Les compositions selon la présente demande peuvent aussi contenir au moins un polymère filmogène hydrophile ou lipophile.

Dans la présente demande, on entend par "polymère filmogène", un polymère apte à former à lui seul ou en présence d'un agent auxiliaire de filmification, un film macroscopiquement continu et adhérent sur les cils, et de préférence un film cohésif, et mieux encore un film dont la cohésion et les propriétés mécaniques sont telles que ledit film peut être isolable et manipulable isolément, par exemple lorsque ledit film est réalisé par coulage sur une surface anti-adhérente comme une surface téflonnée ou siliconnée.

De façon générale, la teneur en "polymère filmogène" des compositions selon la présente demande va de 0,1 à 40 %, de préférence de 0,5 à 30 % et mieux de 1 à 20 % en poids, par rapport au poids total de la composition,

Le polymère filmogène hydrophile peut être un polymère hydrosoluble ou se présenter en dispersion dans un milieu aqueux.

Parmi les polymères filmogènes utilisables dans la composition de la présente invention, on peut citer les polymères synthétiques, de type radicalaire ou de type polycondensat, les polymères d'origine naturelle, et leurs mélanges.

Comme exemples de polymères filmogènes hydrosolubles, on peut citer :
- les protéines comme les protéines d'origine végétale telles que les protéines de blé, de soja ; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères de chitine ou de chitosane anioniques, cationiques, amphotères ou non-ioniques ,
- les gommes arabiques, la gomme de guar, les dérivés du xanthane, la gomme de karaya ;
- les alginates et les carraghénanes ;
- les glycoaminoglycanes, l'acide hyaluronique et ses dérivés ;
- la résine shellac, la gomme de sandaraque, les dammars, les élémis, les copals ;
- l'acide désoxyribonucléïque ;
- les muccopolysaccharides tels les chondroïtines sulfate, et leurs mélanges.

Le polymère filmogène peut être également présent dans la composition sous la forme de particules en dispersion dans une phase aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations Neocryl XK-90^{®}, Neocryl A-1070^{®}, Neocryl A-1090^{®}, Neocryl BT-62^{®}, Neocryl A-1079^{®} et Neocryl A-523^{®} par la société AVECIA-NEORESINS, Dow Latex 432^{®} par la société DOW CHEMICAL, Daitosol 5000 AD^{®} ou Daitosol 5000 SJ^{®} par la société DAITO KASEY KOGYO; Syntran 5760^{®} par la société Interpolymer Allianz Opt^{®} par la société Rohm and Haas ou encore les dispersions aqueuses de polyuréthane vendues sous les dénominations Neorez R-981^{®} et Neorez R-974^{®} par la société AVECIA-NEORESINS, les Avalure UR-405^{®}, Avalure UR-410^{®}, Avalure UR-425^{®}, Avalure UR-450^{®}, Sancure 875^{®}, Avalure UR-445^{®} et Sancure 2060^{®} par la société NOVEON, Impranil 85^{®} par la société BAYER, Aquamere H-1511^{®} par la société HYDROMER ; les sulfopolyesters vendus sous le nom de marque Eastman AQ^{®} par la société Eastman Chemical Products, les dispersions vinyliques comme le Mexomère PAM^{®}, les dispersions aqueuses de polyvinyl acétate comme le "Vinybran^{®}" de la société Nisshin Chemical ou celles commercialisées par la société UNION CARBIDE, les dispersions aqueuses de terpolymère vinyl pyrrolidone, diméthylaminopropyl méthacrylamide et chlorure de lauryldiméthylpropylméthacrylamidoammonium telles que le Styleze W-d'ISP, les dispersions aqueuses de polymères hybrides polyuréthane/polyacryliques telles que celles commercialisées sous les références "Hybridur^{®}" par la société AIR PRODUCTS ou "Duromer^{®}" de NATIONAL STARCH, les dispersions type core/shell : par exemple celles commercialisées par la société ATOFINA sous la référence Kynar (core : fluoré- shell : acrylique) ou encore ceux décrits dans le document US 5 188 899 (core ; silice - shell : silicone) et leurs mélanges.

Le polymère lipophile peut être en solution ou en dispersion dans une phase solvant non aqueuse

Les compositions selon la présente demande peuvent aussi contenir au moins un gélifiant hydrophile, ils peuvent être choisi parmi :
- les homo- ou copolymères d'acides acrylique ou méthacrylique ou leurs sels et leurs esters et en particulier les produits vendus sous les dénominations VERSICOL F^{®} ou VERSICOL K^{®} par la société ALLIED COLLOID, UTRAHOLD 8^{®} par la société CIBA-GEIGY, les acides polyacryliques de type SYNTHALEN K,
- les copolymères d'acide acrylique et d'acrylamide vendus sous la forme de leur sel de sodium sous les dénominations RETEN^{®} par la société HERCULES, les sels de sodium d'acides polyhydroxycarboxyliques vendus sous la dénomination HYDAGEN F^{®} par la société HENKEL,
- les copolymères acide polyacryliques/acrylates d'alkyle de type PEMULEN,
- l'AMPS (Acide polyacrylamidométhyl propane sulfonique neutralisé partiellement à l'ammoniaque et hautement réticulé) commercialisé par la société CLARIANT,
- les copolymères AMPS/acrylamide de type SEPIGEL^{®} ou SIMULGEL^{®} commercialisés par la société SEPPIC, et
- les copolymères AMPS/méthacrylates d'alkyle polyoxyéthylénés (réticulés ou non), et leurs mélanges.
- les polyuréthanes associatifs tels que le polymère C₁₆-OE₁₂₀-C₁₆ de la société SERVO DELDEN (commercialisé sous le nom SER AD FX1100, molécule à fonction uréthanne et poids moléculaire moyen en poids de 1300), OE étant un motif oxyéthyléné, le Rhéolate 205 à fonction urée vendu par la société RHEOX ou encore le Rhéolate 208 ou 204 (ces polymères étant vendus sous forme pure) ou le DW 1206B de chez RHOM & HAAS à chaîne alkyle en C₂₀ et à liaison uréthane, vendu à 20 % en matière active dans l'eau. On peut aussi utiliser des solutions ou dispersions de ces polyuréthanes associatifs notamment dans l'eau ou en milieu hydroalcoolique. A titre d'exemple, de tels polymères on peut citer, le SER AD fx1010, le SER AD FX1035 et le SER AD 1070 de la société SERVO DELDEN, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS, ou bien encore le Borchigel LW 44 de la société BORCHERS,
- et leurs mélanges.

Certains polymères filmogènes hydrosolubles cités plus haut peuvent également jouer le rôle de gélifiant hydrosoluble.

Les gélifiants hydrophiles peuvent être présents dans les compositions selon l'invention en une teneur allant de 0,05 à 40% en poids par rapport au poids total de la composition, de préférence de 0,1 à 20% et mieux de 0,5 à 15% en poids.

Les compositions selon la présente demande peuvent aussi contenir au moins une ou plusieurs huiles ou solvant organique.

Par huile ou solvant organique, on entend un corps non aqueux liquide à température ambiante et pression atmosphérique. L'huile peut être volatile ou non volatile.

Par " huile ou solvant organique volatile", on entend au sens de l'invention tout milieu non aqueux susceptible de s'évaporer au contact des matières kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg). Par "huile non volatile", on entend une huile restant sur les matières kératiniques à température ambiante et pression atmosphérique au moins plusieurs heures et ayant notamment une pression de vapeur inférieure à 10⁻³ mm de Hg (0,13Pa).

L'huile peut être présente dans la composition dans une teneur allant de 0,05 à 30 %, de préférence 0,1 à 15 % en poids par rapport au poids total de la composition. La composition selon l'invention peut comprendre des huiles volatiles et/ou des huiles non volatiles, et leurs mélanges.

Les huiles (ou solvants organiques) volatiles peuvent être des huiles hydrocarbonées, des huiles siliconées, des huiles fluorées ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux "d'Isopars^{®}" ou de "Permetyls^{®}", les esters ramifiés en C₈-C₁₆, le néopentanoate d'iso-hexyle, et leurs mélanges. D'autres huiles hydrocarbonées volatiles comme les distillats de pétrole, notamment ceux vendus sous la dénomination "Shell Solt^{®}" par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 6 centistokes (6.10⁻⁶ m²/s), et ayant notamment de 3 à 6 atomes de silicium, ces silicones comportant éventuellement un ou plusieurs groupes alkyles ou alkoxy ayant de 1 ou 2 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également utiliser des solvants organiques volatils notamment fluorés tels que le nonafluorométhoxy butane ou le perfluorométhylcyclopentane.

Chacune des compositions conformes à l'invention peut également comprendre au moins une huile ou solvant organique non volatile, qui peut être en particulier choisie parmi les huiles hydrocarbonées et/ou siliconées et/ou fluorées non volatiles.

Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C₄ à C₂₄, ces derniers pouvant être linéaires ou ramifiés, saturés ou insaturés ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations de Miglyol 810^{®}, 812^{®} et 818^{®} par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
et leurs mélanges.

Les huiles de silicone non volatiles utilisables dans la composition conforme à l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans les compositions conformes à l'invention sont notamment des huiles fluorosiliconées, des polyéthers fluorés, des silicones fluorées telles que décrit dans le document EP-A-847752.

La teneur en huile ou solvant organique non volatile dans la composition conforme à l'invention va de 0,01 à 30 % en poids, en particulier de 0,1 à 25 % en poids, et mieux de 0,1 à 20 % par rapport au poids total de la composition.

Les compositions conformes à l'invention comprennent également au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

Ces matières colorantes peuvent être présentes en une teneur allant de 0,01 à 30 % en poids par rapport au poids total de la composition.

Les compositions conformes à l'invention peuvent également comprendre au moins une charge.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon^{®} commercialisé sous la dénomination Orgasol^{®} par la société Atochem, de poly-β-alanine et de polyéthylène, les poudres de polymères de tétrafluoroéthylène comme le Téflon^{®}, la lauroyl-lysine, l'amidon, le nitrure de bore, les micro sphères creuses polymériques expansées telles que celles de chlorure de polyvinylidène/acrylonitrile comme celles commercialisées sous la dénomination d'Expancel^{®} par la société Nobel Industrie, les poudres acryliques telles que celles commercialisées sous la dénomination Polytrap^{®} par la société Dow Corning, les particules de polyméthacrylate de méthyle et les microbilles de résine de silicone (Tospearls^{®} de Toshiba, par exemple), le carbonate de calcium précipité, le carbonate et l'hydro-carbonate de magnésium, l'hydroxyapatite, les microsphères de silice creuses (Silica Beads^{®} de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.

On peut également utiliser un composé susceptible de gonfler à la chaleur et notamment des particules thermoexpansibles telles que les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel^{®} 820 DU 40 et Expancel^{®}007WU par la Société AKZO NOBEL.

Les charges peuvent représenter de 0,1 à 25 %, en particulier de 0,2 à 20 % en poids par rapport au poids total de la composition.

Les compositions conformes à l'invention peuvent également comprendre au moins une fibre qui permet une amélioration de l'effet allongeant.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 µm à 10 mm, en particulier de 0,1 mm à 5 mm et plus particulièrement de 0,3 mm à 3,5 mm. Leur section peut être comprise dans un cercle de diamètre allant de 2 nm à 500 µm, en particulier allant de 100 nm à 100 µm et plus particulièrement de 1 µm à 50 µm. Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.

Les fibres utilisables dans la composition de l'invention peuvent être choisies parmi les fibres rigides ou non rigides, elles peuvent être d'origine synthétique ou naturelle, minérales ou organiques.

Par ailleurs, les fibres peuvent être traitées ou non en surface, enrobées ou non, colorées ou non colorées.

A titre de fibres utilisables dans la composition selon l'invention, on peut citer les fibres non rigides telles que les fibres de polyamide (Nylon®) ou les fibres rigides telles que les fibres de polyimide-amide comme celles vendues sous les dénomination KERMEL^{®}, KERMEL TECH^{®} par la société RHODIA ou de poly-(p-phénylène-téréphtalamide) (ou d'aramide) notamment vendues sous la dénomination Kevlar ® par la société DUPONT DE NEMOURS.

Les fibres peuvent êtres présentes dans la composition selon l'invention en une teneur allant de 0,01 % à 10 % en poids, par rapport au poids total de la composition, en particulier de 0,1 % à 5 % en poids, et plus particulièrement de 0,3 % à 3 % en poids.

Les compositions conformes à l'invention peuvent également comprendre au moins un actif cosmétique.

Comme actifs cosmétiques pouvant être utilisés dans les compositions conformes à l'invention, on peut citer notamment les antioxydants, les conservateurs, les parfums, les neutralisants, les émollients, les épaississants, les agents de coalescence, les plastifiants, les hydratants, les vitamines et les filtres en particulier solaires, et leurs mélanges.

Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

De préférence, la composition selon l'invention est non rincée. Avantageusement, la composition est un mascara.

Les exemples suivants sont donnés à titre illustratif de la présente invention, et ne sauraient en limiter la portée.

### Exemples

Les compositions suivantes ont été réalisées. Les compositions 1 à 5 sont des compositions comparatives et les compositions 6 à 8 sont conformes à l'invention.

Les quantités indiquées sont exprimées en pourcentage massique par rapport au poids total de la composition.

| Composition | 1 | 2 | 3 | 4 | 5 | 6 invention | 7 invention | 8 invention |
|---|---|---|---|---|---|---|---|---|
| Cire de carnauba | 3.21 | 3.21 | 3.21 | 3.21 | 3.21 | 3.21 | 3.21 | 3.21 |
| Cire d'abeille | 4.07 | 4.07 | 4.07 | 4.07 | 4.07 | 4.07 | 4.07 | 4.07 |
| Cire de paraffine | 12.86 | 12.86 | 12.86 | 12.86 | 12.86 | 12.86 | 12.86 | 12.86 |
| Hydroxy ethylcellulose | 0.89 | 0.89 | 0.89 | 0.89 | 0.89 | 0.89 | 0.89 | 0.89 |
| Gomme arabique | 3.39 | 3.39 | 3.39 | 3.39 | 3.39 | 3.39 | 3.39 | 3.39 |
| Simethicone | 0.13 | 0.13 | 0.13 | 0.13 | 0.13 | 0.13 | 0.13 | 0.13 |
| Oxyde de fer | 7.14 | 7.14 | 7.14 | 7.14 | 7.14 | 7.14 | 7.14 | 7.14 |
| conservateur | 1 | 1 | 1 | 1 | 1 | 1 | 1 | 1 |
| Steareth-20 (Brij 78P d'Uniqema) | 10 | - | - | 6.66 | 6.66 | - | 4,44 | 4,44 |
| Phosphate de cétyle (Arlatone MAP de Uniqema) | - | 10 | - | - | 3.33 | 6.66 | 2,18 | 2,18 |
| Steareth-2 (Brij 72 d'Uniqema) | - | - | 10 | 3.33 | - | 3.33 | 2,1 | 2,1 |
| Alcool cétylique | | | | | | | - | 2 |
| eau | Qsp 100 | Qsp 100 | Qsp 100 | Qsp 100 | Qsp 100 | Qsp 100 | Qsp 100 | Qsp 100 |

La viscosité de chacune des compositions est mesurée selon le protocole décrit plus haut.

| Résultats | 1 | 2 | 3 | 4 | 5 | 6 invention | 7 invention | 8 invention |
|---|---|---|---|---|---|---|---|---|
| Viscosité (en Pa.s) | **1,4** | **13** | **9,5** | **1,7** | **1,3** | **14,6** | **6,4** | **15,1** |
| Qualité de la dispersion | Moyen -ne | Mauvaise | Mauvaise | Moyen - ne | Mauvaise | Bonne | Bonne | Bonne |
| Couleur du produit final | gris | gris | gris | gris | gris | Noir | Noir | Noir |

Les compositions selon la présente demande permettent l'obtention de mascaras de viscosité satisfaisante et présentant une bonne dispersion des pigments qui assure une teinte noire, telle que recherchée pour ce type de produit. Ces mascaras s'appliquent facilement sur les cils et forment un dépôt chargeant, lisse et homogène

## Revendications

1. Composition cosmétique de revêtement des cils comprenant une phase aqueuse et un système émulsionnant, **caractérisée en ce que** le système émulsionnant contient :
- au moins un phosphate d'alkyle en C₁₀-C₃₀,
- au moins un éther d'alcool gras en C₈-C₂₄ et de polyéthylène glycol, ledit éther comprenant de 1 à 19 motifs d'éthylèneglycol et présentant une HLB < 8 à 25°C, et
- au moins une matière colorante.

2. Composition cosmétique selon la revendication 1, **caractérisée en ce que** le phosphate d'alkyle est choisi parmi les phosphates d'alkyle en C₁₆-C₁₈ et leurs mélanges.

3. Composition cosmétique selon la revendication 2, **caractérisée en ce que** le phosphate d'alkyle est choisi parmi le phosphate de cétyle, le phosphate de stéaryle et le phosphate de cétéaryle, de préférence le tensioactif phosphaté est le phosphate de cétyle.

4. Composition cosmétique selon l'une des revendications précédentes, **caractérisée en ce que** la teneur en phosphate(s) d'alkyle va de 0,1 à 20% en poids, de préférence de 0,5 à 12% en poids par rapport au poids total de la composition.

5. Composition cosmétique selon l'une des revendications précédentes, **caractérisée en ce que** l'éther d'alcool gras en C₈-C₂₄ et de polyéthylène glycol comprenant de 1 à 19 motifs d'éthylèneglycol et présentant une HLB < 8 à 25°C est choisi parmi les éthers d'alcools gras en C₁₆-C₂₂ et de polyéthylène glycol comprenant de 1 à 19 motifs d'éthylèneglycol.

6. Composition cosmétique selon la revendication précédente **caractérisée, en ce que** l'éther d'alcool gras en C₈-C₂₄ et de polyéthylène glycol comprenant de 1 à 19 motifs d'éthylèneglycol et présentant une HLB < 8 à 25°C est le steareth-2.

7. Composition cosmétique selon l'une des revendications précédentes **caractérisée en ce que** la teneur en éther(s) d'alcool gras en C₈-C₂₄ et de polyéthylène glycol comprenant de 1 à 19 motifs d'éthylèneglycol et présentant une HLB < 8 à 25°C va de 0,1% à 20% en poids, par rapport au poids total de la composition, de préférence entre 0,5% à 10% en poids.

8. Composition cosmétique selon l'une des revendications précédentes, **caractérisée en ce que** le système émulsionnant tel que défini à la revendication 1 constitue le système tensioactif principal de la composition cosmétique.

9. Composition cosmétique selon l'une des revendications précédentes, **caractérisée en ce que** le système émulsionnant tel que défini à la revendication 1 constitue l'unique système tensioactif de la composition cosmétique.

10. Composition cosmétique selon l'une des revendications précédentes, caractérisée qu'elle comprend moins de 1%, de préférence moins de 0,5% en poids de triéthanolamine.

11. Composition cosmétique selon l'une des revendications précédentes, **caractérisée en ce qu'**elle est exempte de triéthanolamine.

12. Composition cosmétique selon l'une des revendications précédentes **caractérisée en ce qu'**elle comprend moins de 1%, de préférence moins de 0,5% de en poids de stéarate de triéthanolamine.

13. Composition cosmétique selon l'une des revendications précédentes **caractérisée en ce qu'**elle est exempte de stéarate de triéthanolamine.

14. Composition cosmétique selon l'une des revendications précédentes **caractérisée en ce que** la phase aqueuse est formée d'eau ou d'un mélange d'eau et d'au moins un solvant hydrosoluble.

15. Composition cosmétique selon la revendication précédente **caractérisée en ce que** le solvant hydrosoluble est choisi parmi les monoalcools inférieurs ayant de 1 à 5 atomes de carbone, les glycols ayant de 2 à 8 atomes de carbone

16. Composition cosmétique selon la revendication 14 ou 15 **caractérisée en ce que** la phase aqueuse est présente en une teneur allant de 1 % à 95 % en poids, par rapport au poids total de la composition, de préférence allant de 3 % à 80 % en poids, et préférentiellement allant de 5 % à 60 % en poids.

17. Composition cosmétique selon l'une des revendications précédentes **caractérisée en ce qu'**elle comprend au moins un agent tensioactif additionnel choisi parmi les agents tensioactifs non ioniques, anioniques, cationiques, amphotères ou encore les émulsionnants tensioactifs.

18. Composition cosmétique selon l'une des revendications précédentes **caractérisée en ce qu'**elle comprend un tensioactif additionnel de HLB > 8 à 25°C.

19. Composition cosmétique selon l'une des revendications précédentes **caractérisée en ce qu'**elle comprend un éther d'alcool gras en C₈-C₂₄ et de polyéthylène glycol, ledit éther comprenant de 20 à 1000 motifs d'éthylèneglycol.

20. Composition cosmétique selon l'une des revendications précédentes, **caractérisée en ce qu'**elle comprend du Steareth-20.

21. Composition cosmétique selon l'une des revendications précédentes, **caractérisée en ce qu'**elle comprend un co-tensioactif choisi parmi les alcools gras.

22. Composition cosmétique selon la revendication précédente, **caractérisée en ce que** l'alcool gras comprend de 10 à 30 atomes de carbone, de préférence de 10 à 26 atomes de carbone, mieux de 10 à 24 atomes de carbone et encore mieux de 14 à 22 atomes de carbone.

23. Composition cosmétique selon l'une des revendications précédentes **caractérisée en ce qu'**elle comprend au moins une cire en une teneur allant de 0,1 % à 50% en poids, par rapport au poids total de la composition, de préférence de 1 % à 40% en poids, et préférentiellement allant de 5% à 30% en poids.

24. Composition cosmétique selon l'une des revendications précédentes **caractérisée en ce qu'**elle comprend au moins un polymère filmogène hydrophile ou lipophile et/ou au moins un gélifiant hydrophile.

25. Composition cosmétique selon l'une des revendications précédentes **caractérisée en ce qu'**elle comprend au moins une huile.

26. Composition cosmétique selon l'une des revendications précédentes **caractérisée en ce qu'**elle comprend au moins un additif choisi parmi les charges, les fibres, les antioxydants, les conservateurs, les parfums, les neutralisants, les émollients, les épaississants, les agents de coalescence, les plastifiants, les hydratants, les vitamines et les filtres en particulier les filtres solaires, et leurs mélanges.

27. Procédé de maquillage ou de soin non thérapeutique des cils comprenant l'application sur lesdits cils de la composition selon l'une des revendications précédentes.

28. Utilisation de la composition selon l'une des revendications 1 à 26 pour obtenir un maquillage homogène et/ou volumateur des cils.

29. Utilisation de la composition selon l'une des revendications 1 à 26 à titre de mascara pour le maquillage des cils

## Patentansprüche

1. Kosmetische Wimpernbeschichtungszusammensetzung, umfassend eine wässrige Phase und ein Emulgiersystem, **dadurch gekennzeichnet, dass** das Emulgiersystem
- mindestens ein C₁₀-C₃₀-Alkylphosphat,
- mindestens einen Polyethylenglykol-C₈-C₂₄-fett-alkoholether mit 1 bis 19 Ethylenglykol-Einheiten und einem HLB-Wert < 8 bei 25°C und
- mindestens ein Farbmittel
enthält.

2. Kosmetische Zusammensetzung nach Anspruch 1, **dadurch gekennzeichnet, dass** das Alkylphosphat aus C₁₆-C₁₈-Alkylphosphaten und Mischungen davon ausgewählt ist.

3. Kosmetische Zusammensetzung nach Anspruch 2, **dadurch gekennzeichnet, dass** das Alkylphosphat aus Cetylphosphat, Stearylphosphat und Cetearylphosphat ausgewählt ist und es sich bei dem Phosphat-Tensid vorzugsweise um Cetylphosphat handelt.

4. Kosmetische Zusammensetzung nach einem der vorhergehenden Ansprüche, **dadurch gekennzeichnet, dass** der Gehalt an Alkylphosphat(en) 0,1 bis 20 Gew.-% und vorzugsweise 0,5 bis 12 Gew.-%, bezogen auf das Gesamtgewicht der Zusammensetzung, beträgt.

5. Kosmetische Zusammensetzung nach einem der vorhergehenden Ansprüche, **dadurch gekennzeichnet, dass** der Polyethylenglykol-C₈-C₂₄-fettalkoholether mit 1 bis 19 Ethylenglykol-Einheiten und einem HLB-Wert < 8 bei 25°C aus Polyethylenglykol-C₁₆-C₂₂-fettalkoholethern mit 1 bis 19 Ethylenglykol-Einheiten ausgewählt ist.

6. Kosmetische Zusammensetzung nach dem vorhergehenden Anspruch, **dadurch gekennzeichnet, dass** es sich bei dem Polyethylenglykol-C₈-C₂₄-fettalkoholether mit 1 bis 19 Ethylenglykol-Einheiten und einem HLB-Wert < 8 bei 25°C um Steareth-2 handelt.

7. Kosmetische Zusammensetzung nach einem der vorhergehenden Ansprüche, **dadurch gekennzeichnet, dass** der Gehalt an Polyethylenglykol-C₈-C₂₄-fettalkoholether(n) mit 1 bis 19 Ethylenglykol-Einheiten und einem HLB-Wert < 8 bei 25°C 0,1 bis 20 Gew.-%, bezogen auf das Gesamtgewicht der Zusammensetzung, vorzugsweise 0,5 bis 10 Gew.-%, beträgt.

8. Kosmetische Zusammensetzung nach einem der vorhergehenden Ansprüche, **dadurch gekennzeichnet, dass** das Emulgiersystem gemäß Anspruch 1 das hauptsächliche Tensidsystem der kosmetischen Zusammensetzung darstellt.

9. Kosmetische Zusammensetzung nach einem der vorhergehenden Ansprüche, **dadurch gekennzeichnet, dass** das Emulgiersystem gemäß Anspruch 1 das einzige Tensidsystem der kosmetischen Zusammensetzung darstellt.

10. Kosmetische Zusammensetzung nach einem der vorhergehenden Ansprüche, **dadurch gekennzeichnet, dass** sie weniger als 1 Gew.-% und vorzugsweise weniger als 0,5 Gew.-% Triethanolamin umfasst.

11. Kosmetische Zusammensetzung nach einem der vorhergehenden Ansprüche, **dadurch gekennzeichnet, dass** sie frei von Triethanolamin ist.

12. Kosmetische Zusammensetzung nach einem der vorhergehenden Ansprüche, **dadurch gekennzeichnet, dass** sie weniger als 1 Gew.-% und vorzugsweise weniger als 0,5 Gew.-% Triethanolaminstearat umfasst.

13. Kosmetische Zusammensetzung nach einem der vorhergehenden Ansprüche, **dadurch gekennzeichnet, dass** sie frei von Triethanolaminstearat ist.

14. Kosmetische Zusammensetzung nach einem der vorhergehenden Ansprüche, **dadurch gekennzeichnet, dass** die wässrige Phase aus Wasser oder einer Mischung von Wasser und mindestens einem wasserlöslichen Lösungsmittel gebildet wird.

15. Kosmetische Zusammensetzung nach dem vorhergehenden Anspruch, **dadurch gekennzeichnet, dass** das wasserlösliche Lösungsmittel aus niederen Monoalkoholen mit 1 bis 5 Kohlenstoffatomen und Glykolen mit 2 bis 8 Kohlenstoffatomen ausgewählt ist.

16. Kosmetische Zusammensetzung nach Anspruch 14 oder 15, **dadurch gekennzeichnet, dass** die wässrige Phase in einem Anteil im Bereich von 1 bis 95 Gew.-%, bezogen auf das Gesamtgewicht der Zusammensetzung, vorzugsweise im Bereich von 3 bis 80 Gew.-% und bevorzugt im Bereich von 5 bis 60 Gew.-% vorliegt.

17. Kosmetische Zusammensetzung nach einem der vorhergehenden Ansprüche, **dadurch gekennzeichnet, dass** sie mindestens ein zusätzliches Tensid, das aus nichtionischen, anionischen, kationischen und amphoteren Tensiden oder auch Tensidemulgatoren ausgewählt ist, umfasst.

18. Kosmetische Zusammensetzung nach einem der vorhergehenden Ansprüche, **dadurch gekennzeichnet, dass** sie ein zusätzliches Tensid mit einem HLB-Wert > 8 bei 25°C umfasst.

19. Kosmetische Zusammensetzung nach einem der vorhergehenden Ansprüche, **dadurch gekennzeichnet, dass** sie einen Polyethylenglykol-C₈-C₂₄-fettalkoholether umfasst, wobei der Ether 20 bis 1000 Ethylenglykol-Einheiten umfasst.

20. Kosmetische Zusammensetzung nach einem der vorhergehenden Ansprüche, **dadurch gekennzeichnet, dass** sie Steareth-20 umfasst.

21. Kosmetische Zusammensetzung nach einem der vorhergehenden Ansprüche, **dadurch gekennzeichnet, dass** sie ein Cotensid, das aus Fettalkoholen ausgewählt ist, umfasst.

22. Kosmetische Zusammensetzung nach dem vorhergehenden Anspruch, **dadurch gekennzeichnet, dass** der Fettalkohol 10 bis 30 Kohlenstoffatome, vorzugsweise 10 bis 26 Kohlenstoffatome, besser 10 bis 24 Kohlenstoffatome und noch besser 14 bis 22 Kohlenstoffatome umfasst.

23. Kosmetische Zusammensetzung nach einem der vorhergehenden Ansprüche, **dadurch gekennzeichnet, dass** sie mindestens ein Wachs in einem Anteil im Bereich von 0,1 bis 50 Gew.-%, bezogen auf das Gesamtgewicht der Zusammensetzung, vorzugsweise 1 bis 40 Gew.-% und bevorzugt im Bereich von 5 bis 30 Gew.-% umfasst.

24. Kosmetische Zusammensetzung nach einem der vorhergehenden Ansprüche, **dadurch gekennzeichnet, dass** sie mindestens ein hydrophiles oder lipophiles filmbildendes Polymer und/oder mindestens einen hydrophilen Gelbildner umfasst.

25. Kosmetische Zusammensetzung nach einem der vorhergehenden Ansprüche, **dadurch gekennzeichnet, dass** sie mindestens ein Öl umfasst.

26. Kosmetische Zusammensetzung nach einem der vorhergehenden Ansprüche, **dadurch gekennzeichnet, dass** sie mindestens ein Additiv, das aus Füllstoffen, Fasern, Antioxidantien, Konservierungsstoffen, Duftstoffen, Neutralisationsmitteln, Emollienzien, Verdickungsmitteln, Koaleszenzmitteln, Weichmachern, Feuchtigkeitsspendern, Vitaminen und Schutzmitteln, insbesondere Lichtschutzmitteln, und Mischungen davon ausgewählt ist, umfasst.

27. Nichttherapeutisches Verfahren zum Schminken oder Pflegen der Wimpern, bei dem man auf die Wimpern die Zusammensetzung nach einem der vorhergehenden Ansprüche aufbringt.

28. Verwendung der Zusammensetzung nach einem der Ansprüche 1 bis 26 zum Erhalt eines homogenen und/oder volumengebenden Make-ups auf den Wimpern.

29. Verwendung der Zusammensetzung nach einem der Ansprüche 1 bis 26 als Mascara zum Schminken der Wimpern.

## Claims

1. Cosmetic eyelash coating composition comprising an aqueous phase and an emulsifying system, **characterized in that** the emulsifying system contains:
- at least one C₁₀-C₃₀ alkyl phosphate,
- at least one C₈-C₂₄ fatty alkyl ether of polyethylene glycol, the said ether comprising from 1 to 19 ethylene glycol units and having an HLB < 8 at 25°C, and
- at least one colorant

2. Cosmetic composition according to Claim 1, **characterized in that** the alkyl phosphate is chosen from C₁₆-C₁₈ alkyl phosphates, and mixtures thereof.

3. Cosmetic composition according to Claim 2, **characterized in that** the alkyl phosphate is chosen from cetyl phosphate, stearyl phosphate and cetearyl phosphate, and the phosphate surfactant is preferably cetyl phosphate.

4. Cosmetic composition according to one of the preceding claims, **characterized in that** the content of alkyl phosphate (s) ranges from 0.1% to 20% by weight and preferably from 0.5% to 12% by weight relative to the total weight of the composition.

5. Cosmetic composition according to one of the preceding claims, **characterized in that** the C₈-C₂₄ fatty alkyl ether of polyethylene glycol comprising from 1 to 19 ethylene glycol units and having an HLB < 8 at 25°C is chosen from C₁₆-C₁₈ fatty alkyl ethers of polyethylene glycol comprising from 1 to 19 ethylene glycol units.

6. Cosmetic composition according to the preceding claim, **characterized in that** the C₈-C₂₄ fatty alkyl ether of polyethylene glycol comprising from 1 to 19 ethylene glycol units and having an HLB < 8 at 25°C is Steareth-2.

7. Cosmetic composition according to one of the preceding claims, **characterized in that** the content of C₈-C₂₄ fatty alkyl ether(s) of polyethylene glycol comprising from 1 to 19 ethylene glycol units and having an HLB < 8 at 25°C ranges from 0.1% to 20% by weight and preferably from 0.5% to 10% by weight relative to the total weight of the composition.

8. Cosmetic composition according to one of the preceding claims, **characterized in that** the emulsifying system as defined in Claim 1 constitutes the main surfactant system of the cosmetic composition.

9. Cosmetic composition according to one of the preceding claims, **characterized in that** the emulsifying system as defined in Claim 1 constitutes the sole surfactant system of the cosmetic composition.

10. Cosmetic composition according to one of the preceding claims, **characterized in that** it comprises less than 1% and preferably less than 0.5% by weight of triethanolamine.

11. Cosmetic composition according to one of the preceding claims, **characterized in that** it is free of triethanolamine.

12. Cosmetic composition according to one of the preceding claims, **characterized in that** it comprises less than 1% and preferably less than 0.5% by weight of triethanolamine stearate.

13. Cosmetic composition according to one of the preceding claims, **characterized in that** it is free of triethanolamine stearate.

14. Cosmetic composition according to one of the preceding claims, **characterized in that** the aqueous phase is formed from water or from a mixture of water and of at least one water-soluble solvent.

15. Cosmetic composition according to the preceding claim, **characterized in that** the water-soluble solvent is chosen from lower monoalcohols containing from 1 to 5 carbon atoms and glycols containing from 2 to 8 carbon atoms.

16. Cosmetic composition according to Claim 14 or 15, **characterized in that** the aqueous phase is present in a content ranging from 1% to 95% by weight, preferably ranging from 3% to 80% by weight and preferentially ranging from 5% to 60% by weight relative to the total weight of the composition.

17. Cosmetic composition according to one of the preceding claims, **characterized in that** it comprises at least one additional surfactant chosen from nonionic, anionic, cationic and amphoteric surfactants, or surfactant emulsifiers.

18. Cosmetic composition according to one of the preceding claims, **characterized in that** it comprises an additional surfactant of HLB > 8 at 25°C.

19. Cosmetic composition according to one of the preceding claims, **characterized in that** it comprises a C₈-C₂₄ fatty alkyl ether of polyethylene glycol, the said ether comprising from 20 to 1000 ethylene glycol units.

20. Cosmetic composition according to one of the preceding claims, **characterized in that** it comprises Steareth-20.

21. Cosmetic composition according to one of the preceding claims, **characterized in that** it comprises a co-surfactant chosen from fatty alcohols.

22. Cosmetic composition according to the preceding claim, **characterized in that** the fatty alcohol contains from 10 to 30 carbon atoms, preferably from 10 to 26 carbon atoms, better still from 10 to 24 carbon atoms and even better still from 14 to 22 carbon atoms.

23. Cosmetic composition according to one of the preceding claims, **characterized in that** it comprises at least one wax in a content ranging from 0.1% to 50% by weight, preferably from 1% to 40% by weight and preferentially ranging from 5% to 30% by weight relative to the total weight of the composition.

24. Cosmetic composition according to one of the preceding claims, **characterized in that** it comprises at least one hydrophilic or lipophilic film-forming polymer and/or at least one hydrophilic gelling agent.

25. Cosmetic composition according to one of the preceding claims, **characterized in that** it comprises at least one oil.

26. Cosmetic composition according to one of the preceding claims, **characterized in that** it comprises at least one additive chosen from fillers, fibres, antioxidants, preserving agents, fragrances, neutralizers, emollients, thickeners, coalescers, plasticizers, moisturizers, vitamins and screening agents, in particular sunscreens, and mixtures thereof.

27. Process for making up or for non-therapeutically caring for the eyelashes, comprising the application to the said eyelashes of the composition according to one of the preceding claims.

28. Use of the composition according to one of Claims 1 to 26, for obtaining uniform and/or volumizing makeup on the eyelashes.

29. Use of the composition according to one of Claims 1 to 26, as mascara for making up the eyelashes.
